# EUROPEAN PATENT APPLICATION

(11) **EP 1 323 449 A1**
(43) Date of publication of application: **02.07.2003**
(21) Application number: 01830826.2
(22) Date of filing: 28.12.2001
(51) Int. Cl.: A61M 25/06

(54) **safety butterfly needle for venipuncture**

(71) Applicant: Restelli, Sergio, 00100 Rome (IT); Righi, Nardino, 20047 Brugherio (Milano) (IT); Rossi, Roberto, 20151 Milano (IT)
(72) Inventor: Restelli, Sergio, 00100 Rome (IT); Righi, Nardino, 20047 Brugherio (Milano) (IT); Rossi, Roberto, 20151 Milano (IT)
(74) Representative: Petruzziello, Aldo

(57) **Abstract**

A butterfly needle (100) for venipuncture comprises a body (1) with two outwardly protruding flexible wings (2) foldable by manual gripping by the user from a diverging position to a converging position, and a needle (9) mounted axially in the head (32) of a needle holder which has a tail (8) protruding rearward form said body (1) to couple with the connection of a flexible tube designed to be connected to a syringe or bottle, the needle holder (3) being mounted axially slidable in said body (1) to pass from a forward use position, in which the needle (9) extends forward from said body (1) to be able to perform the venipuncture to a retracted safety position in which the needle (9) is protected inside the body (1), stop means (6, 7) being provided to block the needle holder in said forward position and in said retracted position.

## Description

The present invention refers to a safety butterfly needle for venipuncture.

As is known, butterfly needles for venipuncture are widely available on the market. Said butterfly needles are generally used for intravenous injections, infusions, blood sampling, transfusions and the like. Although specific reference will be made herein to injections, it is to be understood that the butterfly needle according to the invention can also be used for blood sampling and the like.

A butterfly needle generally comprises a needle proper to penetrate into the vein. Said needle is supported integrally by a needle holder or needle carrier on which two flexible fins shaped like butterfly wings are mounted.

The rear end of the needle is connected to a small flexible plastic tube. The tube ends in a wider mouth, which is closed by a stopper. The mouth of the tube is designed to couple with the head of a syringe filled with solution to be injected for intravenous injections or with the head of a container or bottle for intravenous infusions.

Said flexible wings perform a dual function: that is to say, they act as a grip for the operator who must perform the injection and they act as a securing means for securing the needle in place to the patient's skin during the injection. In fact the operator, using the thumb and index finger of his hand, grasps the body of the butterfly needle by the wings, pinching them together, and then inserts the needle into the patients vein with an inclination of about 25-45° with respect to the surface of the patient's skin.

As soon as the needle enters the vein, blood begins to fill the plastic tube connected to the needle. This phenomenon is called "flashback" and confirms that the needle is positioned correctly in the vein. At this point the operator decreases the inclination of the needle and at the same time advances it deeper into the vein, then he spreads the wings which come into contact with the patient's skin blocking the needle in position and finally he uses surgical tape to fix the wings to the patient's skin.

Once the treatment has been completed, the user removes the tape, folds the wings to make them converge and removes the needle from the vein. The assembly of butterfly needle, tube, syringe, bottle etc. cannot be re-used and therefore is sent for disposal. This operation proves extremely dangerous because the butterfly needle remains exposed and this leads to the risk of accidental needle sticks both by the operator responsible for the injection and by the personnel responsible for disposal.

The object of the present invention is to overcome the drawbacks of the prior art by providing a safety butterfly needle that is able to avoid accidental needle sticks.

Another object of the present invention is to provide such a safety butterfly needle that is practical and simple for the user to use.

Yet another object of the invention is to provide such a safety butterfly needle that is economical and simple to make.

These objects are achieved in accordance with the invention with the characteristics listed in appended independent claim 1.

Advantageous embodiments of the invention are apparent from the dependent claims.

The safety butterfly needle for venipuncture according to the invention comprises a substantially cylindrical body with two outwardly protruding flexible wings bendable by manual gripping by the user from a diverging to a converging position.

A needle is mounted axially in the head of a needle holder or needle carrier coupled to the body, so that the needle protrudes axially forward from the body. The needle holder has a tail protruding rearward from the body to couple with the connection of a flexible tube destined to be connected to a syringe or to a bottle for injection of a solution or for collection of blood.

The main characteristic of the invention is that the needle holder is mounted axially slidable in the body to pass from a forward use position, in which the needle protrudes forward from the body to be able to perform the venipuncture, to a retracted safety position in which the needle is protected inside the body. The butterfly needle according to the invention comprises stopping and locking means able to lock the needle holder in said forward use position to be able to perform the venipuncture and in said retracted safety position to prevent the needle from protruding from the body.

The advantages of the butterfly needle according to the invention are apparent. In fact, once the injection has been performed the needle is protected in a safe position, thus avoiding accidental injuries.

Further characteristics of the invention will be made clearer by the detailed description that follows, referring to a purely exemplary and therefore non-limiting embodiment thereof, illustrated in the appended drawings in which:
Figure 1 is a top plan view of a butterfly needle according to the invention, in which the needle is covered by a needle cap shown partly in section;
Figure 2 is a side view of the butterfly needle, taken in the direction of the arrow A of Figure 1;
Figure 3 is a front view of the butterfly needle, taken from the left-hand side of Figure 1;
Figure 4 is a rear view of the butterfly needle, taken from the right-hand side of Figure 1;
Figure 5 is an exploded view of the butterfly needle according to the invention, in which the body is shown in axial section;
Figure 6 is a perspective view of the locking lever of the butterfly needle according to the invention alone, removed from the body;
Figure 7 is an axial sectional view taken along sectional plane VII-VII of Figure 1, in which the body is shown in section and the needle holder in a side view.

Figure 8 is a sectional view taken along sectional plane VIII-VIII of Figure 7;

Figure 9 is a sectional view, like Figure 8, illustrating the butterfly needle according to the invention in a safety position, in which the locking lever is shown with a dotted line in the unlocked position.

The butterfly needle according to the invention, denoted as whole with reference numeral 100, is described with the aid of the figures.

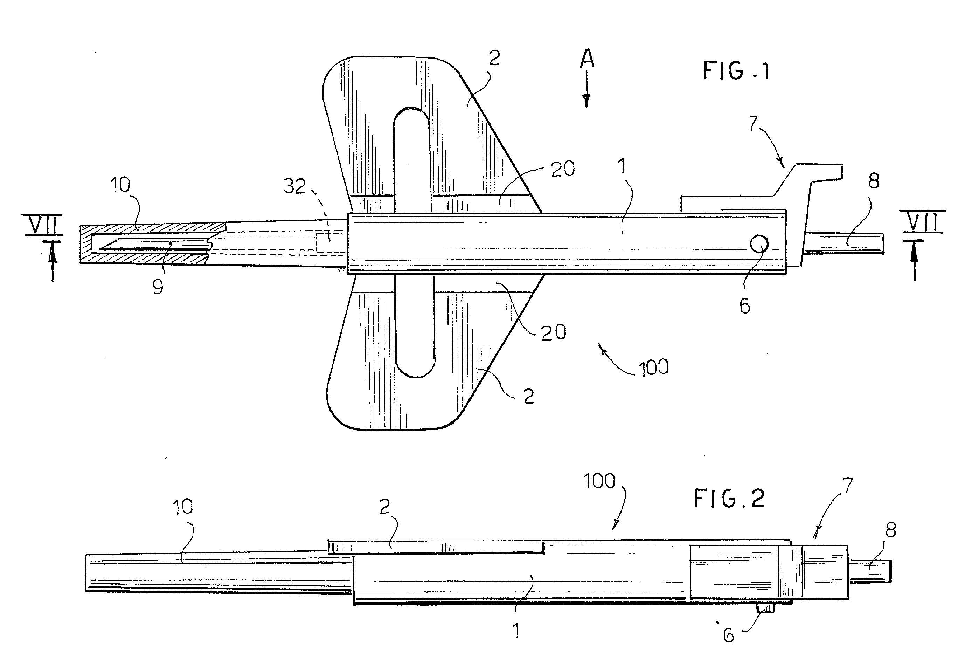

With reference for now to Figures 1-4, the butterfly needle 100 comprises a main body 1, substantially cylindrical in shape, on which two flexible wings 2 are mounted. The wings 2 are mounted in the front part of the body 1 and protrude in opposite directions with respect to the axis of the body. Near the body 1 the wings 2 have longitudinal grooves 20 which act as a fold line to allow the wings 2 to be folded or bent along the grooves 20. In this manner the wings 2, bending along the fold line 20, can pass from an open position, shown in the figures, to a closed position, in which they converge, drawing nearer to each other so that they can be grasped by the user.

A needle 9 is mounted axially in the head 32 of a needle holder 3 which is mounted in the body 1 so that the needle 9 protrudes forward from the body 1 to perform the injection. The needle 9 is covered by a needle cap 10 that snap engages in the head 53 of the needle holder. The needle holder 3 has a tail 8 that protrudes rearward from the body 1 and is able to couple with a special connection of a flexible tube (not shown in the figures) intended to be connected to a syringe or to a bottle containing the solution to be injected or designed to receive the blood that is drawn.

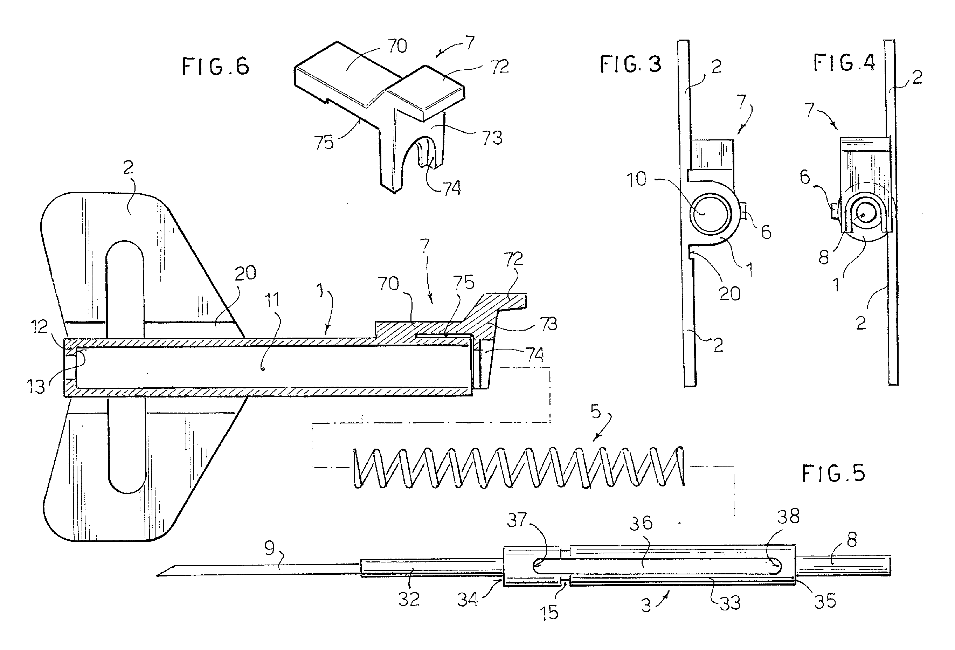

As shown in Figure 5, the body 1 is substantially cylindrical and is hollow on the inside, providing a cylindrical chamber 11 open at the front and rear. In the front end, the body 1 has an annular collar 12 protruding radially inward so as to define an annular abutment surface 13.

In its rear part, on the outside, the body 1 has a locking lever 7. As better illustrated in Figure 6, the locking lever 7 comprises a substantially rectangular flexible plate 70 fixed at one end thereof to the body 1. In the figures, the flexible plate 70 is shown with one end integral with the body 1 and a longitudinal cut 75 that separates the remaining part of the flexible plate 70 from the body 1. In this manner radial removal of the plate 70 from the body 1 and its subsequent elastic return onto the body 1 are allowed.

The plate 70 continues with a raised part 72 able to be operated by the operator and an abutment plate 73 disposed at right angles with respect to the plate 70, so as to protrude at the rear end of the body 1. The abutment plate 73 has a forked shape, substantially like an upturned U, defined by a central groove 74 able to surround part of the side wall of the tail 8 of the needle holder which protrudes rearward from the body 1.

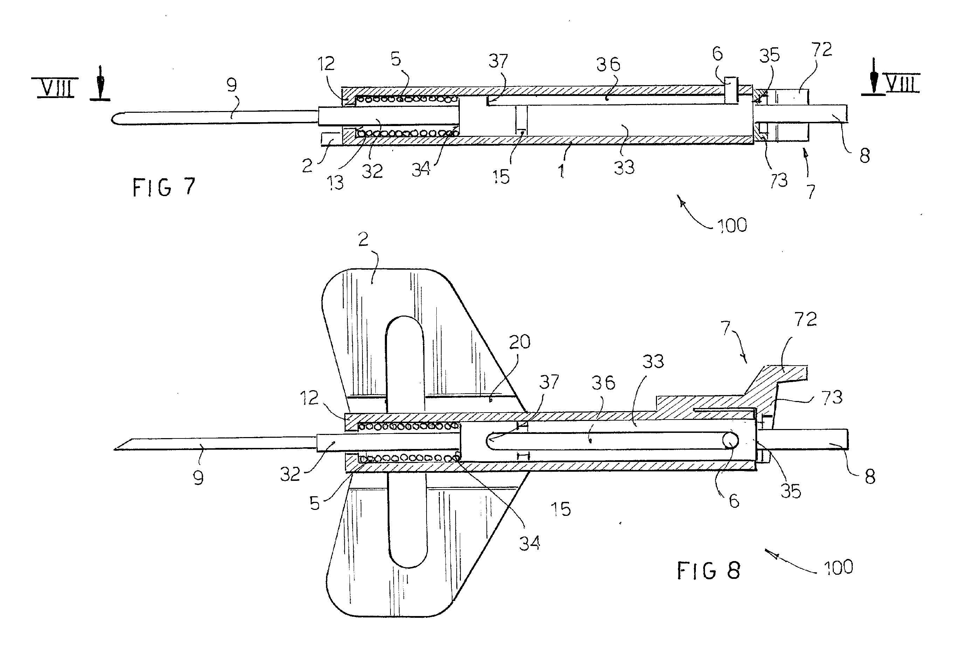

As better shown in Figure 7, the body 1 has in its rear part a stop pin 6 which protrudes radially inside the chamber 11 of the body 1. The stop pin 6 is at an angular distance of about 90° from the locking lever 7.

Returning to Figure 5, the needle holder 30 has a substantially cylindrical body, hollow on the inside, having an axial channel open at the front and rear and communicating with the inner channel of the needle 9. The body of the needle holder 3 comprises a head 32 and a tail 8 having a smaller diameter than a central part 33. In this manner a front annular abutment surface 34 and a rear annular abutment surface 35 are defined at the ends of the central part 33 of the needle holder. The outside diameter of the central part 33 of the needle holder is equal to or slightly smaller than the inside diameter of the body 1 and the outside diameter of the head 32 of the needle holder is equal to or slightly smaller than the inside diameter of the collar 12 of the front part of the body 1.

In the side wall of the central part 33, near the front abutment surface 37 of the longitudinal groove 36, an annular groove 15, which reduces the diameter of the central part 33 of the needle holder 3, is formed. Naturally, for the established objects, instead of the annular groove 15, two opposite notches can be provided, to receive the arms of the fork of the locking lever 7, as will be better described below.

The needle 9 is axially and tightly mounted in the head 32 of the needle holder, so that the channel of the needle communicates with the inner channel of the needle holder.

On the outer surface of the central part 33 of the needle holder a longitudinal groove 36 is provided that extends for almost the entire length of the central part 33, so as to define a front abutment surface 37 and a rear abutment surface 38. The length of the groove 36 is slightly greater than the length of the needle 9.

Assembly of the butterfly needle 100 according to the invention will be described below with reference also to Figures 7 and 8.

A helicoidal spring 5 having an outside diameter slightly smaller than the inside diameter of the body 1 is inserted into the chamber 11 from the rear end of the body 1. Subsequently, again from the rear end of the body 1, the needle holder 3 is inserted into the chamber 11. During said operations the locking lever 7 is kept raised so as not to interfere with insertion of the spring 5 and of the needle holder 3. Owing to the presence of the stop pin 6, the needle holder is forcibly inserted or else the stop pin 6 of the body 1 is initially raised or removed and after insertion of the needle holder it is fixed to the body 1 so that its end engages in the groove 36, in abutment against the rear abutment surface 38 of the groove 36.

Consequently, the head 32 of the needle holder axially enters into the spring 5 and exits partially from the collar 12 of the front end of the body 1. In this manner, one end of the spring 5 is in abutment against the abutment surface 13 of the collar 12 of the body 1 and the other end of the spring 5 is in abutment against the front abutment surface 34 of the central part 33 of the needle holder.

In this manner, by pushing the needle holder axially, the spring 5 is compressed between the abutment surface 13 of the collar 12 of the body 1 and the front abutment surface 34 of the central part 33 of the needle holder, until the rear abutment surface 35 of the central part 33 of the needle holder is level with the rear part of the body 1. At this point the locking lever 7 is released and returns elastically onto the body 1 and the fork-shaped stop plate 73 of the locking lever abuts against the tail 8 of the needle holder. In this situation the rear abutment surface 35 of the central part 33 of the needle holder abuts against the stop plate 73 of the locking lever 7, preventing axial rearward movement of the needle holder 3 against the action of the spring 5 which is under compression. The axial forward movement of the needle holder is avoided by the pin 6 which abuts against the rear abutment surface 38 of the longitudinal groove of the needle holder.

The needle 9 can be pre-assembled in the head of the needle holder 3 or else it can be mounted later given that part of the head 32 of the needle holder protrudes forward from the body 1. In this situation the butterfly needle 100 is ready to be used.

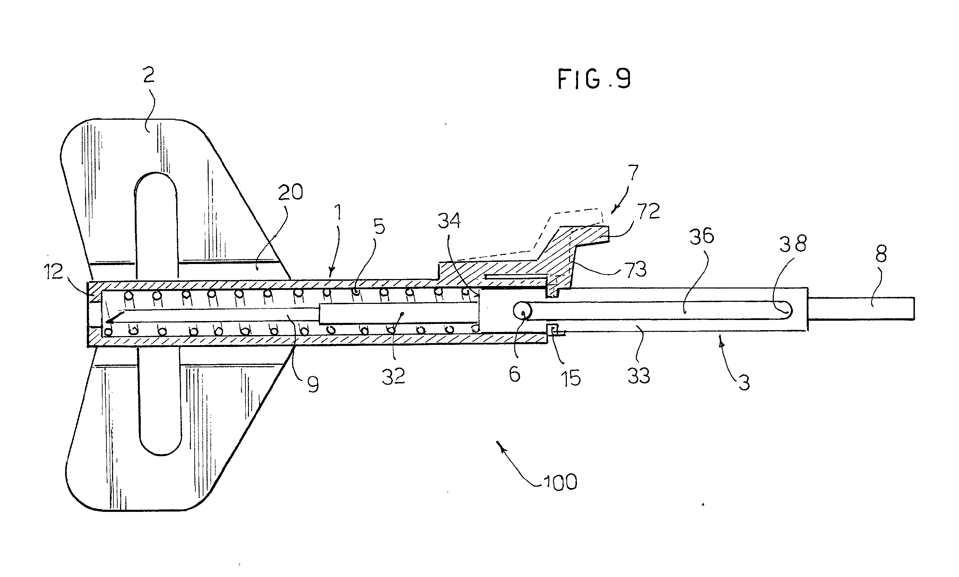

Operation of the safety device of the butterfly needle 100 is described below with reference to Figure 9. Once the injection has been carried out and the needle 9 has been removed from the vein, the operator operates the locking lever 7, making it withdraw from the body 1. Because of the small size of the lever 7, a fingernail must normally be used to perform this operation, as normally it is not sufficient to operate the lever with a finger.

Consequently the rear abutment surface 35 of the central part 33 of the needle holder is no longer retained by the stop plate 73 of the locking lever. Thus through the action of the spring which is released, the needle holder 3 is pushed axially rearward until the front abutment surface 37 of the groove 36 of the needle holder abuts against the stop pin 6, stopping the stroke of the needle holder.

In this situation, the needle 9 is protected inside the body 1, avoiding the risk of accidental needle sticks. Meanwhile the locking lever 7 has elastically returned onto the body 1, consequently fork-shaped stop plate 73 of the locking lever engages in the annular groove 15 formed in the central part 33 of the needle holder which is situated outside the body 1, near the rear end of the body 1.

In this manner, the needle holder is firmly restrained in the safety position. In fact axial rearward movement of the needle holder is prevented by the stop pin 6 which is in abutment against the rear abutment surface 38 of the longitudinal groove 36 and axial forward movement of the needle holder is prevented by the stop plate 73 of the locking lever which is engaged inside the annular groove 15 of the needle holder.

In the present embodiment of the invention a locking lever 7 has been described in the form of a plate having one end integral with the body 1. However, the locking lever 7 can consist simply of a removable cover which snap engages in the body 1 and in the tail 8 of the needle holder to retain the needle holder. Alternatively, the locking lever 7 can be integral or hinged to the tail of the needle holder and removably engageable with the body 1.

Clearly, even if not shown in the figures, other locking means can be provided such as to lock the needle holder 3 in a safe position, avoiding axial forward movements of the needle holder. Said locking means can be elastic tongues provided in the groove 36 of the needle holder to abut against the stop pin 36, when the latter is in abutment against the front abutment surface 37 of the groove 36. Alternatively, said locking means can be elastic tongues provided in the body 1 and protruding inward to abut against a ledge of the needle holder 3.

Numerous variations and modifications of detail within the reach of a person skilled in the art can be made to the present embodiment of the invention, without departing from the scope of the invention as set forth by the appended claims.

## Claims

1. A butterfly needle (100) for venipuncture comprising
- a substantially cylindrical body (1) with two outwardly extending flexible wings (2) bendable by manual gripping by the user from a diverging position to a converging position, and
- a needle (9) mounted axially in the head (32) of a needle holder (3) coupled to said body (1) so that the needle (9) protrudes axially forward from said body (1), said needle holder having a tail (8) protruding rearward from said body (1) to couple with the connection of a flexible tube intended to be connected to a syringe or bottle,
**characterized in that**
said needle holder (3) is axially slidably mounted inside said body (1) to pass from a forward use position, in which the needle (9) protrudes forward from the body (1) to be able to perform the injection, to a retracted safety position in which the needle (9) is protected inside said body (1), stopping and locking means (6, 7) able to lock said needle holder in said forward position and in said retracted position being provided.

2. A butterfly needle (100) according to claim 1, **characterized in that** spring means (5) are disposed inside said body (1), between an abutment surface (13) of said body (1) and an abutment surface (34) of said needle holder (3), said spring means (5) being loaded when said needle holder (3) is situated in the forward use position.

3. A butterfly needle (100) according to claim 1 or 2, **characterized in that** said stop means comprise a stop pin (6) protruding radially toward the inside of said body (1) to engage in a longitudinal groove (36) formed in the side surface of said needle holder (3), said longitudinal groove being defined by a front abutment surface (37) and a rearward abutment surface (38), said stop pin (6) being in abutment against said rearward abutment surface (38), when the needle holder is in the forward use position, and against said front abutment surface (37), when the needle holder is in the retracted safety position.

4. A butterfly needle (100) according to any one of the preceding claims, **characterized in that** said locking means comprise a locking lever (7), constrained to said body (1) and removably engageable with said needle holder (3).

5. A butterfly needle (100) according to claim 4, **characterized in that** said locking lever (7) is elastically constrained to said body (1), so as to bend elastically in an unlocking position in which it allows retraction of the needle holder (3) into its retracted safety position, and to return elastically into a locking position in which it locks the needle holder (3) in its forward use position and in its retracted safety position.

6. A butterfly needle (100) according to claim 5, **characterized in that** said locking lever (7) has a fork-shaped stop wall (73) that abuts against the rear part of the needle holder (3), when the needle holder is in its forward use position, and engages in a seat (15) formed in the outer side wall of said needle holder, when the needle holder is in its retracted safety position.

7. A butterfly needle (100) according to claim 4, **characterized in that** said locking lever comprises a cover removably engageable in said body (1) and in said needle holder (3) to lock the needle holder in its forward use position.

8. A butterfly needle (100) according to any one of claims 4 to 7, **characterized in that** said needle holder (3) comprises a substantially cylindrical central part (33) having a greater diameter than said head (32) and said tail (8) in order to define a front annular abutment surface (34) able to abut against one end of said spring means (5) and a rear annular abutment surface (35) able to abut against said locking lever (7).

9. A butterfly needle (100) according to claim 8, **characterized in that** said seat (15) is an annular groove formed on the outer side surface of said central part (33) of the needle holder.

10. A butterfly needle (100) according to claim 8 or 9, **characterized in that** said longitudinal groove (36) is formed on the outer side surface of said central part (33) of the needle holder and said longitudinal groove (36) has a length equal to or greater than the length of the portion of needle (9) protruding from the body.
